# EUROPEAN PATENT APPLICATION

(11) **EP 3 714 716 A1**
(43) Date of publication of application: **30.09.2020**
(21) Application number: 19166313.7
(22) Date of filing: 29.03.2019
(51) Int. Cl.: A24F 47/00, A61M 11/04, A61M 15/06

(54) **AEROSOL DELIVERY DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

The present disclosure provides an aerosol delivery device for a smoking substitute system, the aerosol delivery device comprising a device airflow path extending from at least one inlet of the device to an outlet of the device and a vaporiser for vaporising a vaporisable liquid. The device airflow path circumvents the vaporiser.

## Description

### Field of the Invention

The present invention relates to an aerosol delivery device, and, more particularly, but not exclusively, to an aerosol delivery device for a smoking substitute system.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful byproducts. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems, which may also be known as electronic nicotine delivery systems, may include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. Some smoking substitute devices are designed to resemble a traditional cigarette and are cylindrical in form with a mouthpiece at one end. Other smoking substitute devices do not generally resemble a cigarette (for example, the smoking substitute device may have a generally box-like form). There are a number of different categories of smoking substitute systems, each utilising a different smoking substitute approach. A smoking substitute approach corresponds to the manner in which the substitute system operates for a user.

One approach is the so-called "vaping" approach, in which a vaporisable liquid, typically referred to (and referred to herein) as "e-liquid", is heated by a heating device to produce an aerosol vapour which is inhaled by a user. An e-liquid typically includes a base liquid as well as nicotine and/or a flavourant. The resulting vapour therefore also typically contains nicotine and/or a flavourant. The base liquid may include propylene glycol and/or vegetable glycerine.

A typical vaping substitute device includes a mouthpiece, a power source (typically a battery), a tank or liquid reservoir for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute systems, which typically have a sealed tank and heating element. The tank is pre-filled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another example vaping smoking substitute system is the blu PRO™ e-cigarette. The blu PRO™ e cigarette is an open system device which includes a main body, a (refillable) tank, and a mouthpiece. The main body and tank are physically and electrically coupled together by screwing one to the other. The mouthpiece and refillable tank are physically coupled together by screwing one into the other, and detaching the mouthpiece from the refillable tank allows the tank to be refilled with e-liquid. The device is activated by a button on the main body. When the device is activated, electrical energy is supplied from the power source to a vaporiser, which heats e-liquid from the tank to produce a vapour which is inhaled by a user through the mouthpiece.

In prior art systems, some of the unvaporised e-liquid passes through the wick and to the mouthpiece. This may result in unvaporised e-liquid passing into the user's mouth, which may be unpleasant for the user. Further, leakage occurs due to leakage paths present between the components of the consumable.

The present disclosure has been devised in the light of the above considerations.

### Summary of the Invention

At its most general, the present invention relates to an aerosol delivery device in which an airflow path through the aerosol delivery device circumvents a vaporiser.

According to a first aspect, there is provided an aerosol delivery device for a smoking substitute system, the aerosol delivery device comprising a device airflow path extending from at least one inlet of the device to an outlet of the device, and a vaporiser for vaporising a vaporisable liquid, wherein the device airflow path circumvents the vaporiser.

In this way, the device airflow path does not pass through the vaporiser. Therefore, the possibility of unvaporised liquid from the vaporiser being entrained in the airflow, and thus through the outlet of the device and into the mouth of a user, is reduced or eliminated.

The terms "transversely" and "transverse" are used herein in relation to components of the device to describe a direction that is substantially perpendicular to the axial (longitudinal) direction of the device.

The device has an upper end comprising the outlet and a lower end comprising the at least one inlet. The device has a longitudinal axis which extends between the upper and lower ends. The term "downstream" used herein is intended to refer to a longitudinal direction of the device towards the outlet. The term "upstream" used herein is intended to refer to a longitudinal direction of the device away from the outlet.

Optional features of the present disclosure will now be set out. These are applicable singly or in any combination with any aspect of the present disclosure.

The vaporiser may be disposed within a vaporising chamber. In these embodiments, the device airflow path comprises at least one inlet airflow path extending from the at least one inlet within a respective inlet channel to the vaporising chamber, and at least one chamber airflow path extending from the channel through the chamber. As the airflow passes through the vaporising chamber, vaporised liquid in the vaporising chamber (which has been vaporised by the vaporiser) may be entrained in the airflow within the chamber airflow path for delivery to the outlet of the device, and therefore to the user.

The or each inlet may be provided on the lowermost surface of the device, e.g. on the lowermost surface of a base portion of the device provided at the lower end of the device.

The or each inlet channel (and the or each inlet airflow path) may extend (e.g. in a generally longitudinal direction) through the base portion of the aerosol delivery device. The or each inlet channel may extend from the respective inlet of the device to a respective inlet channel opening within the vaporising chamber. Thus, the/each inlet airflow path extends from the respective inlet of the device, through the base portion and through the inlet channel opening into the vaporising chamber.

In some embodiments, the or each inlet channel opening (and the or each inlet on the lowermost surface of the device/base portion) is offset from the central longitudinal axis of the aerosol delivery device e.g. off-set in the front to rear direction of the device (which is perpendicular to both the transverse and longitudinal direction).

Where there are two inlet airflow paths, the inlet channel openings (and each inlet on the lowermost surface of the device) may be spaced from each other in the front to rear direction of the device. They may be equally spaced from the central longitudinal axis of the aerosol delivery device on either side of the central longitudinal axis in a front to rear direction. They may be (transversely) aligned with each other in the front to rear direction of the device.

The/each inlet channel opening may be offset from the vaporiser within the vaporising chamber in the front to rear direction of the device. The/each inlet channel opening may be offset from the vaporiser within the vaporising chamber in the longitudinal direction of the device. The opening of the/each inlet channel in the vaporising chamber may be axially downstream of the vaporiser (i.e. closer to the outlet of the device).

In this way, airflow in the inlet airflow path may enter the vaporising chamber downstream of the vaporiser, which may further help to reduce the amount of un-vaporised liquid entrained in the chamber airflow path towards the outlet of the device.

The vaporiser may be transversely elongated e.g. it may comprise a transversely elongated wick and a heating element. The vaporiser may be positioned so that it overlies the axial centre of the base portion (e.g. it extends transversely to intersect the longitudinal axis of the device).

The opening(s) of the or each inlet channel may be elongated in the transverse direction such that it/they extend substantially parallel to the vaporiser (i.e. the wick of the vaporiser). Accordingly, the or each inlet channel may have a transversely elongated (e.g. substantially rectangular) transverse cross-sectional profile. The cross-sectional profile/area of the inlet channel may be substantially uniform between the respective inlet and channel opening. In this way, the airflow within the inlet channel is substantially laminar.

The lowermost (upstream) surface of the base portion may have a generally rectangular profile with opposing transverse edges spaced by opposing front and rear edges (where the front to rear direction of the device extends perpendicular to both the longitudinal and transverse directions).

The or each inlet on the lowermost surface of the base portion may be elongated in the transverse direction such that it/they extend parallel to the front and rear edges of the lowermost surface of the base portion.

The aerosol delivery device may comprise a tank (reservoir) for containing the vaporisable liquid (e.g. an e-liquid) with the vaporiser being in fluid communication with the tank. The e-liquid may, for example, comprise a base liquid and e.g. nicotine. The base liquid may include propylene glycol and/or vegetable glycerine.

The tank may be defined by a tank housing. At least a portion of the tank housing may be translucent. For example, the tank may comprise a window to allow a user to visually assess the quantity of e-liquid in the tank. The tank may be referred to as a "clearomizer" if it includes a window, or a "cartomizer" if it does not.

The base portion may be a base insert defining the inlet(s). The base insert may be inserted into an open lower end of the tank so as to seal against an inside surface of the tank housing. The base insert may comprise an inner, longitudinally-extending sleeve that defines wall(s) of the vaporising chamber. The base insert may be configured to support the vaporiser within the vaporising chamber. The base insert may define the inlet channels. The base insert may be formed of silicone.

The vaporising chamber may comprise opposing parallel side walls that are substantially parallel to the longitudinal axis of the device and are spaced by front and rear walls of the chamber, and a downstream wall extending transversely between the side walls (and front to rear between the front and rear walls). The walls of the vaporising chamber (e.g. the front and rear walls) may have at least one step formed therein, such that a portion of each (front/rear) wall is substantially perpendicular to the longitudinal axis of the device. The step(s) may be provided downstream of the vaporiser within the vaporising chamber. The opening of the/each inlet channel may be formed in the step(s) of the wall(s) of the vaporising chamber (i.e. the opening of the/each inlet channel may be formed in the step portion of the wall perpendicular to the longitudinal axis of the device).

The aerosol delivery device may comprise a passage extending to the outlet of the device e.g. at a mouthpiece of the aerosol delivery device. The passage may extend from a passage opening in the vaporising chamber to the outlet. In this respect, a user may draw fluid (e.g. air) from the inlet, through the inlet channel(s) and through the vaporising chamber into the passage opening and through the passage by inhaling at the outlet (i.e. using the mouthpiece).

The passage may comprise passage walls extending within the tank such that the tank may surround the passage. The passage opening may be formed in the downstream wall of the vaporising chamber. The inner sleeve of the base insert may seal against the passage walls.

The wick may comprise a porous material. The wick may be elongate and extend transversely across the vaporising chamber between wall(s) (e.g. side walls) of the vaporising chamber. The wick may also comprise one or more portions in contact with liquid stored in the tank. For example, opposing transverse ends of the wick may protrude into the tank and a central portion (between the ends) may extend across the vaporising chamber. Thus, fluid may be drawn (e.g. by capillary action) along the wick, from the reservoir to the central portion of the wick.

The filament may be wound about the central portion of the wick. In operation, the power source may supply electricity to (i.e. apply a voltage across) the filament so as to heat the filament. This may cause liquid stored in the wick (i.e. drawn from the tank) to be heated so as to form a vapour and become entrained in the device airflow path. This vapour may subsequently cool to form an aerosol in the passage.

The aerosol delivery device may be in the form of a consumable.

In a second aspect, there is provided a smoking substitute system comprising: the aerosol delivery device of the first aspect; and a main body comprising a power source, wherein the power source supplies power to the vaporiser.

The consumable may be configured for engagement with the main body (i.e. so as to form a closed smoking substitute system). For example, the consumable may comprise components of the system that are disposable, and the main body may comprise non-disposable or non-consumable components (e.g. power supply, controller, sensor, etc.) that facilitate the delivery of aerosol by the consumable. In such an embodiment, the aerosol former (e.g. vaporisable e-liquid) may be replenished by replacing a used consumable with an unused consumable.

The main body and the consumable may be configured to be physically coupled together. For example, the consumable may be at least partially received in a recess of the main body, such that there is an interference fit (e.g. snap engagement) between the main body and the consumable. Alternatively, the main body and the consumable may be physically coupled together by screwing one onto the other, or through a bayonet fitting.

Thus, the aerosol delivery device may comprise one or more engagement portions for engaging with a main body. In this way, the lower end of the aerosol delivery device (e.g. the base portion) may be coupled with the main body, whilst the upper end of the aerosol delivery device may define a mouthpiece of the smoking substitute system.

The power source may be electrically connected (or connectable) to the vaporiser of the aerosol delivery device when engaged with the main body. The power source may be a battery (e.g. a rechargeable battery). A connector in the form of e.g. a USB port may be provided for recharging this battery.

The consumable may comprise an electrical interface for interfacing with a corresponding electrical interface of the main body. A pair of contact pins may comprise the electrical interface for interfacing with a corresponding electrical interface of the main body. One or both of the electrical interfaces may include one or more electrical contacts. Specifically, downstream ends of the contact pins may provide the electrical contacts. Thus, when the main body is engaged with the consumable, the electrical interface may be configured to transfer electrical power from the power source to the heating element of the consumable. The electrical interface may also be used to identify the aerosol delivery device from a list of known types. For example, the consumable may have a certain concentration of nicotine and the electrical interface may be used to identify this. The electrical interface may additionally or alternatively be used to identify when a consumable is connected to the main body.

The main body may comprise an interface, which may, for example, be in the form of an RFID reader, a barcode or QR code reader. This interface may be able to identify a characteristic (e.g. a type) of a consumable engaged with the main body. In this respect, the consumable may include any one or more of an RFID chip, a barcode or QR code, or memory within which is an identifier and which can be interrogated via the interface.

The aerosol delivery device or main body may comprise a controller, which may include a microprocessor. The controller may be configured to control the supply of power from the power source to the vaporiser of the aerosol delivery device (e.g. via the electrical contacts). A memory may be provided and may be operatively connected to the controller. The memory may include non-volatile memory. The memory may include instructions which, when implemented, cause the controller to perform certain tasks or steps of a method.

The main body or aerosol delivery device may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor (i.e. airflow sensor) may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the vaporiser in response to a detected puff. That is, the aerosol delivery device may be configured to be activated when a puff is detected by the puff sensor. The puff sensor may form part of the consumable or the main body.

In an alternative embodiment the device may be a non-consumable device in which an aerosol former (e.g. e-liquid) of the system may be replenished by re-filling the tank of the device (rather than replacing the consumable). In this embodiment, the consumable described above may instead be a non-consumable component that is integral with the main body. Thus the device may comprise the features of the main body described above. In this embodiment, the only consumable portion may be e-liquid contained in the tank of the device. Access to the tank (for re-filling of the e-liquid) may be provided via e.g. an opening to the tank that is sealable with a closure (e.g. a cap).

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1A is a front schematic view of a smoking substitute system;
Figure 1B is a front schematic view of a main body of the system;
Figure 1C is a front schematic view of a consumable of the system;
Figure 2A is a schematic of the components of the main body;
Figure 2B is a schematic of the components of the consumable;
Figure 3 is a section view of the consumable;
Figure 4A is a perspective view of a base insert of the consumable;
Figure 4B is an alternative perspective view of the base insert of Figure 4A; and
Figure 4C is a section view of the base insert of Figure 4A.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

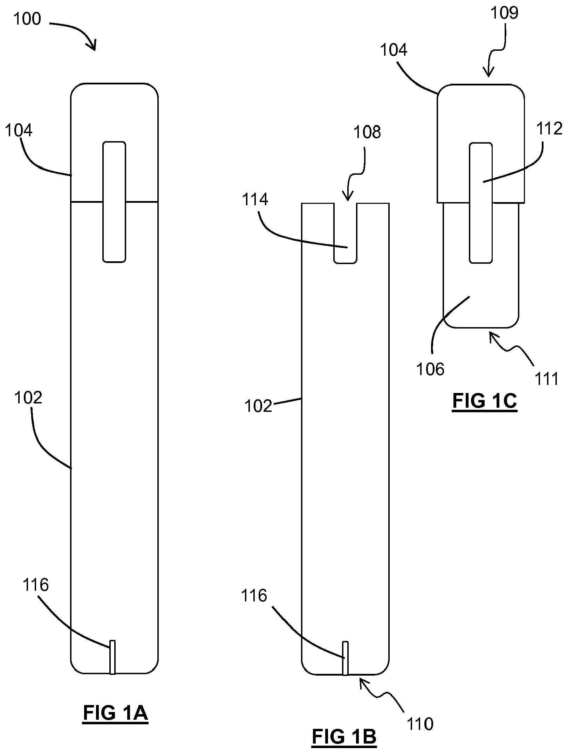

Figure 1A shows a first embodiment of a smoking substitute system 100. In this example, the smoking substitute system 100 includes a main body 102 and an aerosol delivery device in the form of a consumable 104. The consumable 104 may alternatively be referred to as a "pod", "cartridge" or "cartomizer". It should be appreciated that in other examples (i.e. open systems), the main body may be integral with the consumable such that the aerosol delivery device incorporates the main body. In such systems, a tank of the aerosol delivery device may be accessible for refilling the device.

In this example, the smoking substitute system 100 is a closed system vaping system, wherein the consumable 104 includes a sealed tank 106 and is intended for single-use only. The consumable 104 is removably engageable with the main body 102 (i.e. for removal and replacement). Figure 1A shows the smoking substitute system 100 with the main body 102 physically coupled to the consumable 104, Figure 1B shows the main body 102 of the smoking substitute system 100 without the consumable 104, and Figure 1C shows the consumable 104 of the smoking substitute system 100 without the main body 102.

The main body 102 and the consumable 104 are configured to be physically coupled together by pushing the consumable 104 into a cavity at an upper end 108 of the main body 102, such that there is an interference fit between the main body 102 and the consumable 104. In other examples, the main body 102 and the consumable may be coupled by screwing one onto the other, or through a bayonet fitting.

The consumable 104 includes a mouthpiece (not shown in Figure 1A, 1B or 1C) at an upper end 109 of the consumable 104, and one or more air inlets (not shown) in fluid communication with the mouthpiece such that air can be drawn into and through the consumable 104 when a user inhales through the mouthpiece. The tank 106 containing e-liquid is located at the lower end 111 of the consumable 104.

The tank 106 includes a window 112, which allows the amount of e-liquid in the tank 106 to be visually assessed. The main body 102 includes a slot 114 so that the window 112 of the consumable 104 can be seen whilst the rest of the tank 106 is obscured from view when the consumable 104 is inserted into the cavity at the upper end 108 of the main body 102.

The lower end 111 of the main body 102 also includes a light 116 (e.g. an LED) located behind a small translucent cover. The light 116 may be configured to illuminate when the smoking substitute system 100 is activated. While not shown, the consumable 104 may identify itself to the main body 102, via an electrical interface, RFID chip, or barcode.

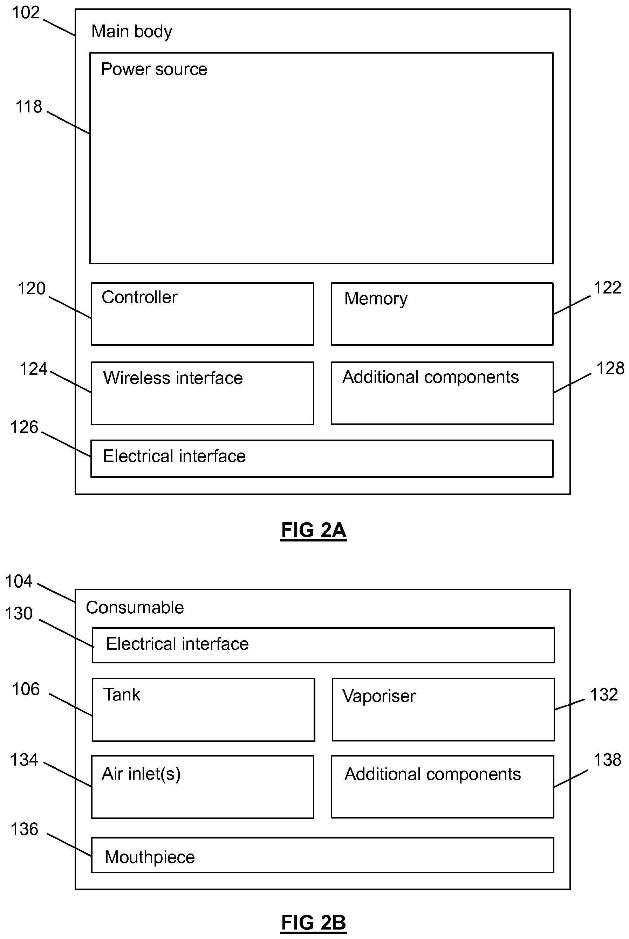

Figures 2A and 2B are schematic drawings of the main body 102 and consumable 104. As is apparent from Figure 2A, the main body 102 includes a power source 118, a controller 120, a memory 122, a wireless interface 124, an electrical interface 126, and, optionally, one or more additional components 128.

The power source 118 is preferably a battery, more preferably a rechargeable battery. The controller 120 may include a microprocessor, for example. The memory 122 preferably includes non-volatile memory. The memory may include instructions which, when implemented, cause the controller 120 to perform certain tasks or steps of a method.

The wireless interface 124 is preferably configured to communicate wirelessly with another device, for example a mobile device, e.g. via Bluetooth®. To this end, the wireless interface 124 could include a Bluetooth® antenna. Other wireless communication interfaces, e.g. WiFi®, are also possible. The wireless interface 124 may also be configured to communicate wirelessly with a remote server.

The electrical interface 126 may be located in a base of the aperture in the upper end 108 of the main body 102. When the main body 102 is physically coupled to the consumable 104, the electrical interface 126 is configured to transfer electrical power from the power source 118 to the consumable 104 (i.e. upon activation of the smoking substitute system 100).

The electrical interface 126 may be configured to receive power from a charging station when the main body 102 is not physically coupled to the consumable 104 and is instead coupled to the charging station. The electrical interface 126 may also be used to identify the consumable 104 from a list of known consumables. For example, the consumable 104 may be a particular flavour and/or have a certain concentration of nicotine (which may be identified by the electrical interface 126). This can be indicated to the controller 120 of the main body 102 when the consumable 104 is connected to the main body 102. Additionally, or alternatively, there may be a separate communication interface provided in the main body 102 and a corresponding communication interface in the consumable 104 such that, when connected, the consumable 104 can identify itself to the main body 102.

The additional components 128 of the main body 102 may comprise the light 116 discussed above.

The additional components 128 of the main body 102 may also comprise a charging port (e.g. USB or micro-USB port) configured to receive power from the charging station (i.e. when the power source 118 is a rechargeable battery). This may be located at the lower end 110 of the main body 102. Alternatively, the electrical interface 126 discussed above may be configured to act as a charging port configured to receive power from the charging station such that a separate charging port is not required.

The additional components 128 of the main body 102 may, if the power source 118 is a rechargeable battery, include a battery charging control circuit, for controlling the charging of the rechargeable battery. However, a battery charging control circuit could equally be located in the charging station (if present).

The additional components 128 of the main body 102 may include a sensor, such as an airflow (i.e. puff) sensor for detecting airflow in the smoking substitute system 100, e.g. caused by a user inhaling through a mouthpiece 136 of the consumable 104. The smoking substitute system 100 may be configured to be activated when airflow is detected by the airflow sensor. This sensor could alternatively be included in the consumable 104. The airflow sensor can be used to determine, for example, how heavily a user draws on the mouthpiece or how many times a user draws on the mouthpiece in a particular time period.

The additional components 128 of the main body 102 may include a user input, e.g. a button. The smoking substitute system 100 may be configured to be activated when a user interacts with the user input (e.g. presses the button). This provides an alternative to the airflow sensor as a mechanism for activating the smoking substitute system 100.

As shown in Figure 2B, the consumable 104 includes the tank 106, an electrical interface 130, a vaporiser 132, one or more air inlets 134, a mouthpiece 136, and one or more additional components 138.

The electrical interface 126 of the main body 102 and an electrical interface 130 of the consumable 104 are configured to contact each other and thereby electrically couple the main body 102 to the consumable 104 when the lower end 110 of the consumable 104 is inserted into the upper end of the main body 102 (as shown in Fig. 1A). In this way, electrical energy (e.g. in the form of an electrical current) is able to be supplied from the power source 118 in the main body 102 to the vaporiser 132 in the consumable 104.

The vaporiser 132 is configured to heat and vaporise e-liquid contained in the tank 106 using electrical energy supplied from the power source 118. As will be described further below, the vaporiser 132 includes a heating filament and a wick. The wick draws e-liquid from the tank 106 and the heating filament heats the e-liquid to vaporise the e-liquid.

The one or more air inlets 134 are preferably configured to allow air to be drawn into the smoking substitute system 100, when a user inhales through the mouthpiece 136. When the consumable 104 is physically coupled to the main body 102, the air inlets 134 receive air, which flows to the air inlets 134 along a gap between the main body 102 and the lower end 110 of the consumable 104.

In operation, a user activates the smoking substitute system 100, e.g. through interaction with a user input forming part of the main body 102 or by inhaling through the mouthpiece 136 as described above. Upon activation, the controller 120 may supply electrical energy from the power source 118 to the vaporiser 132 (via electrical interfaces 126, 130), which may cause the vaporiser 132 to heat e-liquid drawn from the tank 106 to produce a vapour which is inhaled by a user through the mouthpiece 136.

An example of one of the one or more additional components 138 of the consumable 104 is an interface for obtaining an identifier of the consumable 104. As discussed above, this interface may be, for example, an RFID reader, a barcode, a QR code reader, or an electronic interface which is able to identify the consumable. The consumable 104 may, therefore include any one or more of an RFID chip, a barcode or QR code, or memory within which is an identifier and which can be interrogated via the electronic interface in the main body 102.

It should be appreciated that the smoking substitute system 100 shown in figures 1A to 2B is just one exemplary implementation of a smoking substitute system. For example, the system could otherwise be in the form of an entirely disposable (single-use) system or an open system in which the tank is refillable (rather than replaceable).

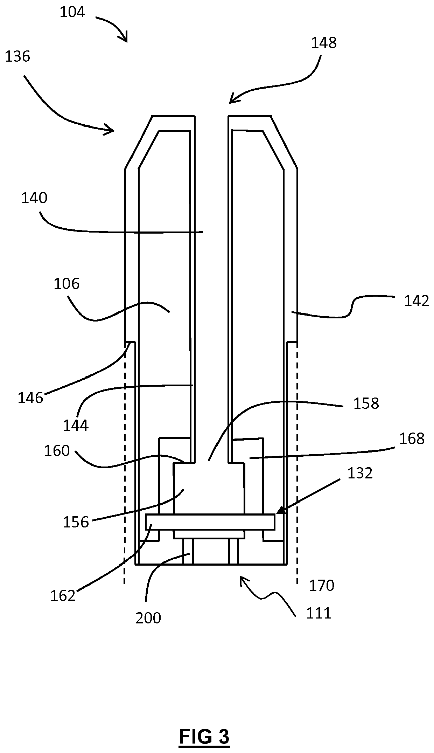

Figure 3 is a section view of the consumable 104 described above. The consumable 104 comprises a tank 106 for storing e-liquid, a mouthpiece 136 and a passage 140 extending along a longitudinal axis of the consumable 104. In the illustrated embodiment the passage 140 is in the form of a tube having a substantially circular transverse cross-section (i.e. transverse to the longitudinal axis). The tank 106 surrounds the passage 140, such that the passage 140 extends centrally through the tank 106.

A tank housing 142 of the tank 106 defines an outer casing of the consumable 104, whilst a passage wall 144 defines the passage 140. The tank housing 142 extends from the lower end of the consumable 111 to the mouthpiece 136 at the upper end 109 of the consumable 104. At the junction between the mouthpiece 136 and the tank housing 142, the mouthpiece 136 is wider than the tank housing 142, so as to define a lip 146 that overhangs the tank housing 142. This lip 146 acts as a stop feature when the consumable 104 is inserted into the main body 102 (i.e. by contact with an upper edge of the main body 102).

The tank 106, the passage 140 and the mouthpiece 136 are integrally formed with each other so as to form a single unitary component. This component may be formed by way of an injection moulding process and, for example, may be formed of a thermoplastic material such as polypropylene.

Although not immediately apparent from the figures, the tank housing 142 tapers, such that the thickness of the tank housing 142 decreases in a downward direction away from the mouthpiece 136. This means that, aside from a small number of indents (which provide physical connection between the consumable 104 and the main body 102), the thickness of the tank housing 142 decreases with increasing distance away from the mouthpiece 136. In particular, the tank housing 142 tapers in this way, because internal and external surfaces of the tank housing 142 are angled with respect to the downward direction away from the mouthpiece 136. This tapering assists in forming the tank housing 142 and passage wall 144 as a single (i.e. unitary) component.

The mouthpiece 136 comprises a mouthpiece aperture 148 defining an outlet of the passage 140.

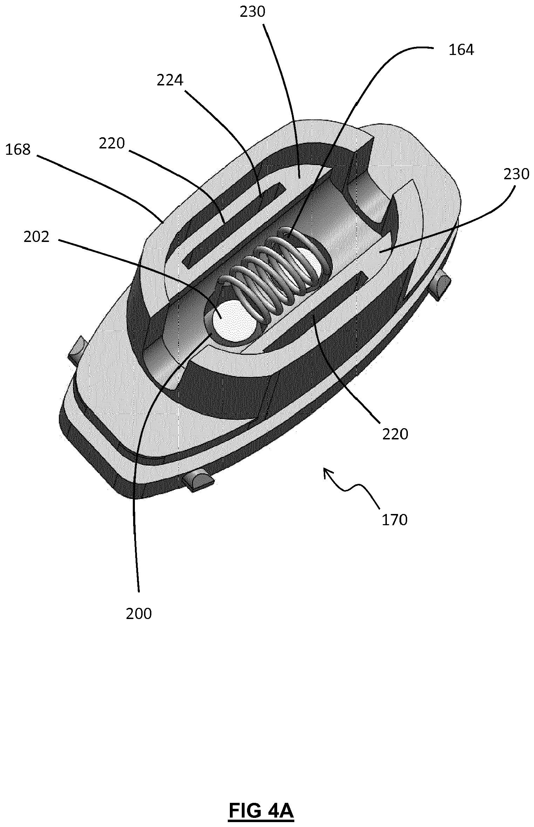

The vaporiser 132 is located in a vaporising chamber 156 of the consumable 104. The vaporising chamber 156 is downstream of the air inlets 134 (discussed later with references to figures 4A-C) of the consumable 104 and is fluidly connected to the mouthpiece aperture 148 (i.e. outlet) by the passage 140. In particular, the passage 140 extends between the mouthpiece aperture 148 and an opening 158 from the chamber 156. This opening 158 is formed in a downstream (i.e. upper) wall 160 of the chamber 156.

The lower end 111 (i.e. base) of the consumable 104 that connects with the main body 102 is defined by a base insert 170. The base insert 170 is inserted into an open lower end of the tank 106 so as to seal against an internal surface of the tank housing 142.

The vaporiser 132 comprises a porous wick 162 and a heater filament 164 (not shown in Figure 3, but described in more detail below in relation to Figures 4A-C) coiled around the porous wick 162. The wick 162 extends transversely across the chamber 156 between sidewalls of the chamber 156 which form part of an inner sleeve 168 of the base insert 170.

The inner sleeve 168 projects into the tank 106 and seals with the passage 140 (around the passage wall 144) so as to separate the chamber 156 from the e-liquid in the tank 106. Transverse ends of the wick 162 project into the tank 106 so as to be in contact with the e-liquid in the tank 106. In this way, e-liquid is transported along the wick 162 (e.g. by capillary action) to a central portion of the wick 162 that is exposed to airflow through the chamber 156. The transported e-liquid is heated by the heater filament 164 (when activated e.g. by detection of inhalation), which causes the e-liquid to be vaporised and to be entrained in air flowing within the vaporising chamber 156. This vaporised liquid may cool to form an aerosol in the passage 140, which may then be inhaled by a user.

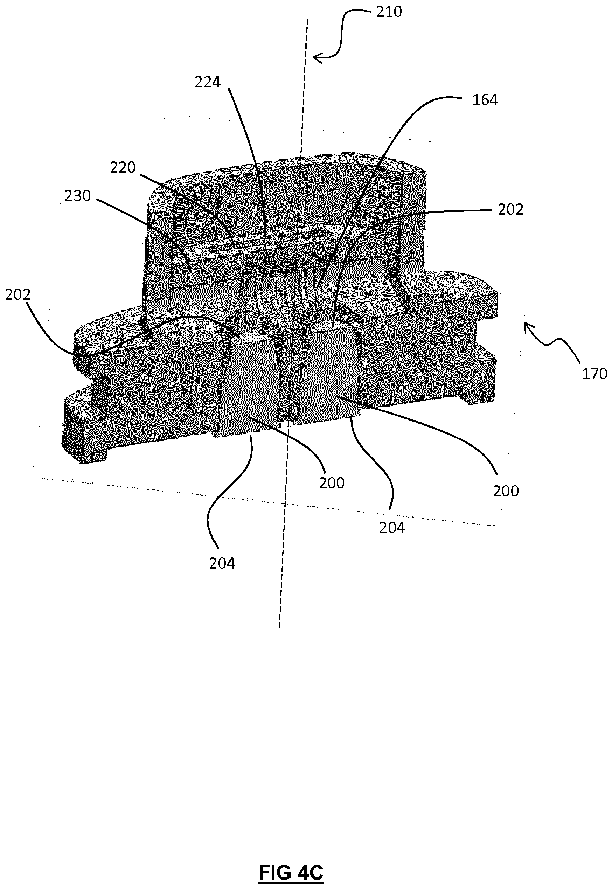

Figure 4A illustrates the base insert 170 of the consumable 104. Figure 4A also illustrates the coiled heater filament 164 but does not show the wick 162. A pair of contact pins 200 having a circular cross section in the longitudinal direction are embedded in the base portion 170. As is more clearly shown in Figure 4C, the contact pins 200 extend through the base portion 170 in a direction substantially parallel to the longitudinal axis of the consumable 104.

An upper (downstream) face 202 of each of the pair of contact pins 200 is electrically connected to respective ends of the heater filament 164. The upper face 202 of each of the pair of contact pins 200 is also physically connected to respective ends of the heater filament 164. The upper faces 202 of the contact pins 200 may be connected to the heater filament 164 by crimping, welding or compressing.

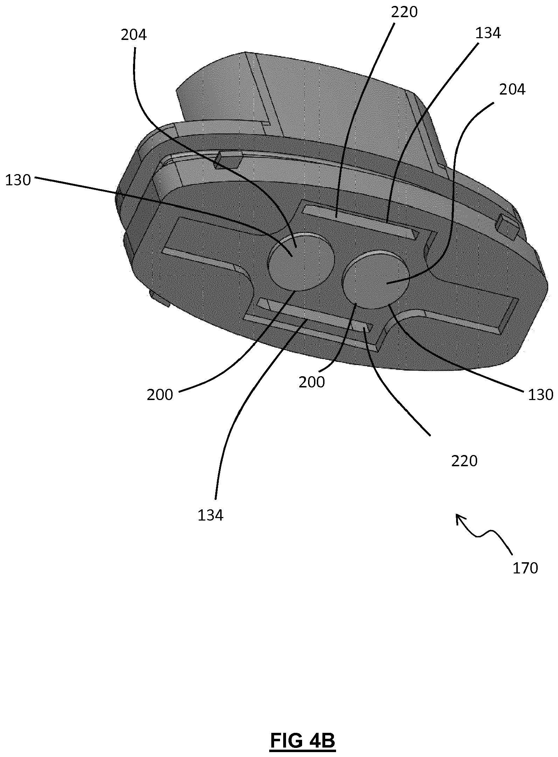

As shown in Figure 4B, a lower (upstream) face 204 of each of the contact pins 200 comprises the electrical interface 130 for interfacing with the corresponding electrical interface 126 on the main body 102. Thus, when the consumable 104 is engaged with the main body 102, the lower faces 204 of the contact pins 200 contact corresponding electrical contacts on the main body 102. As the main body electrical contacts are electrically connected to the power source 118, power can be supplied by the main body 102, via the contact pins 200, to the filament 164 in order to heat the filament 164.

The contact pins 200 are aligned with each other in a transverse direction perpendicular to the longitudinal direction of the device. The contact pins 200 are also transversely aligned parallel to the transversely extending wick 162.

Furthermore, as shown more clearly in Figure 4C, the connection between the upper face 202 of each contact pin 200 and the heater filament 164 is located upstream of the heater filament 164 and wick 162.

As shown in Figure 4C, the heating filament 164 and the wick 162 are positioned to overlie the axial centre of the base insert 170. Accordingly, the heating filament 164 and wick 162 are centrally positioned in the consumable 104 in the transverse plane relative to the longitudinal axis of the consumable 104, such that the central longitudinal axis 210 of the consumable 104 intersects the heating filament 164 and wick 162.

Each contact pin 200 is spaced in the transverse direction from the central longitudinal axis 210 of the consumable 104. Specifically, each contact pin 200 is spaced from the central longitudinal axis 210 of the consumable 104 by a same distance on either side of the central longitudinal axis 210.

Each of the contact pins 200 is substantially cylindrical. However, as shown in Figures 4A-4C, each contact pin 200 tapers towards the upper face 202, which is connected to the heating filament 164.

The contact pins 200 may be formed from a metal/metal alloy with high electrical conductivity. The contact pins 200 may be formed from one or more of silver, copper, gold, platinum, palladium, tungsten, nickel, graphite, molybdenum, for example.

As previously mentioned, the heating filament 164 and wick 162 are positioned within a vaporising chamber 156. As shown in Figures 4A-C, a pair of inlet channels 220 extend through the base insert 170 into the vaporising chamber 156. The inlet channels extend in a generally longitudinal direction of the device. They allow air to flow through the base insert 170 from device inlets 134 at the lowermost surface 111 of the consumable 104 (i.e. lowermost surface of the base portion 170), through openings 224 into the vaporising chamber 156. Thus, by drawing on the mouthpiece 136, a user may draw air through the device inlets 134, the inlet channels 220, the vaporising chamber 156, the passage 140, and out through the outlet 148 in the mouthpiece 136.

In Figures 4A-4C, the two openings 224 of the inlet channels 220 are transversely offset from the central longitudinal axis 210 of the consumable 104 on either side of the central longitudinal axis 210 in the front to back direction. The two openings 224 of the inlet channels 220 are equally spaced from the central longitudinal axis 210 of the aerosol delivery device on either side of the central longitudinal axis 210. They are aligned with each other in the front to rear direction.

The openings 224 of the inlet channels 220 are formed in perpendicular stepped portions 230 in the front and rear walls of the vaporising chamber 156. The stepped portions 230 in the front and rear walls, and therefore the openings 224 of the inlet channels 220, are axially downstream of the heating filament 164 and the wick 162.

The openings 224 of the inlet channels 220 are elongated in the transverse direction such that they extend substantially parallel to the transversely-extending wick 162. The device inlets 134 at the lowermost surface 111 of the base portion 170 are also elongated in the transverse direction such that they extend substantially parallel to the transverse axis aligning the lower faces 204 of the contact pins 200 on the lowermost surface of the base portion.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery device for a smoking substitute system, the aerosol delivery device comprising:
a device airflow path extending from at least one inlet of the device to an outlet of the device; and
a vaporiser for vaporising a vaporisable liquid, wherein the device airflow path circumvents the vaporiser.

2. The aerosol delivery device of claim 1, wherein the vaporiser is disposed within a vaporising chamber, and the device airflow path comprises a chamber airflow path extending through the vaporising chamber, the chamber airflow path circumventing the vaporiser.

3. The aerosol delivery device of claim 2, wherein the device airflow path comprises at least one inlet airflow path extending within a respective inlet channel from the at least one inlet to a respective channel opening within the vaporising chamber.

4. The aerosol delivery device of claim 3, wherein the inlet channel(s) extend(s) in a generally longitudinal direction through a base portion of the aerosol delivery device.

5. The aerosol delivery device of claim 3 or claim 4, wherein the channel opening(s) is/are axially downstream of the vaporiser.

6. The aerosol delivery device of any one of claims 3-5, wherein the inlet channel opening(s) is/are offset from a central longitudinal axis of the aerosol delivery device.

7. The aerosol delivery device of any one of claims 3-6, wherein the inlet channel(s) has/have a transversely elongated transverse cross-sectional profile.

8. The aerosol delivery device of any of one claims 3-7, wherein the inlet channel opening(s) is/are elongated in a transverse direction.

9. The aerosol delivery device of claim 8, wherein the vaporiser is transversely elongated, and the inlet channel opening(s) extend(s) substantially parallel to the vaporiser and offset in a front to rear direction.

10. The aerosol delivery device of any one of claims 3-9, wherein the device airflow path comprises a pair of inlet airflow paths extending within respective inlet channels from a respective inlet to a respective inlet channel opening within the vaporising chamber, and wherein the inlet channel openings are aligned with each other in a front to rear direction perpendicular to the transverse direction and the longitudinal direction.

11. The aerosol delivery device of claim 10, wherein the inlet channel openings are equally spaced from the central longitudinal axis of the aerosol delivery device in the front to rear direction.

12. A smoking substitute system comprising:
the aerosol delivery device of any one of the preceding claims; and
a main body comprising a power source, wherein the power source supplies power to the vaporiser.
